# EUROPEAN PATENT APPLICATION

(11) **EP 4 789 647 A1**
(43) Date of publication of application: **12.08.2026**
(21) Application number: 24874677.8
(22) Date of filing: 03.10.2024
(51) Int. Cl.: A61F 13/494, A61F 13/49, A61F 13/514, A61F 13/515

(54) **DISPOSABLE WEARABLE ARTICLE**

(30) Priority: 06.10.2023 JP 2023174162
(71) Applicant: Daio Paper Corporation, Ehime 799-0492 (JP)
(72) Inventor: UJIIE, Kodai, Sakura-shi, Tochigi 329-1411 (JP)
(74) Representative: Meissner Bolte Partnerschaft mbB
(86) International application number: PCT/JP2024/035406
(87) International publication number: WO 2025/075077

(57) **Abstract**

[Problem] To improve the fit in fold-over type standing gathers.

[Solution] The above problem is solved by a standing gather including a front fallen section 67F and a rear fallen section 67B each including a first portion 66a extending from a starting end 61 toward the center in a width direction WD, and a second portion 66b extending from a distal end of this first portion 66a outwardly in the width direction WD. A standing portion 68 includes a third portion 66c continuous from the first portions 66a and a fourth portion 66d continuous from the second portions 66b. A second gather elastic member 63b extends along a boundary between the third portion 66c and the fourth portion 66d. No gather elastic member 63 is present in a region from the second gather elastic member 63b toward the starting end 61. A gather waterproof sheet 64 made of a waterproof resin film covering substantially the entire third portion 66c is fixed to the standing portion 68, thereby forming a high-stiffness region, while the fourth portion 66d is free of the gather waterproof sheet 64 and has fewer sheet layers than the third portion 66c, so that bending is less likely to occur between the second gather elastic member 63b and the starting end 61.

## Description

### Technical Field

The present invention relates to a disposable wearing article with standing gathers on both sides.

### Background Art

Disposable wearing articles such as pad-type, tapetype, pull-up, and other types of disposable diapers and sanitary pads are commonly provided with standing gathers to prevent side leakage of bodily exudates. The gathers are provided on both sides in the width direction of the front surface to stand up from their starting ends that extend along the front-back direction (e.g., see PTL 1 and PTL 2).

There are various types of standing gathers, many of which have the following basic configuration. Namely, the standing gather includes: a main body extending from the starting end toward the center in the width direction; a front fallen section formed by fixing a front end portion of the main body in a fallen state; a rear fallen section formed by fixing a rear end portion of the main body in a fallen state; a non-fixed standing portion located between the front and rear fallen sections of the main body; and a plurality of gather elastic members extending along the front-back direction and spaced apart in the width direction in the standing portion.

Preferably, the standing gathers of the disposable wearing article make contact with the body surface at a shallower angle and over a wider area, and stand up higher in a narrower surface-occupation area (the surface area of the main body in the unfolded state of the disposable wearing article). From this point of view, it is preferable that the front fallen section and the rear fallen section each include a first portion extending from the starting end toward the center in the width direction and a second portion extending from the distal end of this first portion outwardly in the width direction, and that the standing portions include a third portion continuous from the first portion of the front fallen section to the first portion of the rear fallen section and a fourth portion continuous from the second portion of the front fallen section and the second portion of the rear fallen section (hereinafter also referred to as a fold-over type).

However, in such fold-over type standing gathers, the boundary between the first and second portions is fixed in the fallen sections, whereas the third and fourth portions that continue from them and form the standing portion are not. Accordingly, depending on the way the article is worn, the standing portion may fold at positions other than the intended boundary between the third and fourth portions, and may bend at several spaced points, resulting in poor fit of the fourth portion.

### Citation List

### Patent Literature

PTL 1: Japanese Patent Application Publication No. 2021-69864
PTL 2: Japanese Patent No. 7252084

### Summary of Invention

### Technical Problem

Accordingly, a main object of the present invention is to improve the fit of fold-over type standing gathers. Solution to Problem

The above problem is solved by the absorbent article described below.

### <First aspect>

A disposable wearing article with a standing gather provided on either side in a width direction of a front surface to stand up from a starting end thereof that extends along a front-back direction,
the standing gather including: a main body extending from the starting end toward a central region in the width direction; a front fallen section formed by fixing a front end portion of the main body in a fallen state; a rear fallen section formed by fixing a rear end portion of the main body in a fallen state; a non-fixed standing portion located between the front fallen section and the rear fallen section of the main body; and a plurality of gather elastic members extending along the front-back direction and spaced apart in the width direction in the standing portion,
the front fallen section and the rear fallen section each including a first portion extending from the starting end toward the central region in the width direction and a second portion extending from a distal end of this first portion outwardly in the width direction, the standing portion including a third portion continuous from the first portion of the front fallen section to the first portion of the rear fallen section and a fourth portion continuous from the second portion of the front fallen section and the second portion of the rear fallen section,
the gather elastic members including a first gather elastic member attached to a distal end of the fourth portion opposite to the third portion, and a second gather elastic member attached along a boundary between the third portion and the fourth portion,
the standing gather being free of the gather elastic member in a region from the second gather elastic member toward the starting end,
the standing portion being provided with a gather waterproof sheet made of a waterproof resin film covering the third portion, fixedly attached to extend at least over an entire length in the front-back direction of the standing portion,
the gather waterproof sheet extending, from a position located within 5 mm from the second gather elastic member toward the starting end, to at least the starting end,
a fixed region between the gather waterproof sheet and the standing portion extending from a position located within 5 mm from an end of the gather waterproof sheet on a side of the second gather elastic member toward the starting end, to at least the starting end,
the standing gather further including a gather nonwoven fabric covering the third portion and the fourth portion, the fourth portion being free of the gather waterproof sheet and having fewer sheet layers than the third portion.

### (Operational effect)

The standing gather of this disposable wearing article includes a first gather elastic member attached to the distal end of the fourth portion opposite to the third portion, and a second gather elastic member attached along the boundary between the third portion and the fourth portion. The standing gather is free of the gather elastic member in a region from the second gather elastic member toward the starting end. As a result, the fourth portion stands up at a low angle and makes surface contact with the body surface, and the third portion rises up higher. Substantially the entire third portion of the third and fourth portions constitutes a high-stiffness region to which a waterproof resin film having particularly high stiffness (the gather waterproof sheet) is bonded. Since a region near the distal end of the third portion is lifted by the second gather elastic member, a fold-back of the standing portion is unlikely to occur at locations other than along the second gather elastic member, and bending is particularly unlikely to occur between the second gather elastic member and the starting end. Namely, the third portion not only ensures a certain stand-up height, but also provides shape retainability, in addition to high waterproof properties given by the gather waterproof sheet. On the other hand, the fourth portion is free of the waterproof resin film with high stiffness (gather waterproof sheet), and has fewer sheet layers than the third portion, so that it feels softer (less stiff) when it contacts the skin.

### <Second aspect>

The disposable wearing article according to the first aspect, including an absorbent and
a liquid-permeable topsheet provided on a front side of the absorbent,
wherein the topsheet includes a topsheet extension extending out to either side in the width direction of the absorbent,
the topsheet extension including a topsheet fold-over portion folded back at the starting end onto the front side,
the topsheet fold-over portion extending from the starting end to a position located within ± 5 mm in the width direction relative to the end of the gather waterproof sheet on the side of the second gather elastic member, and being fixed to the gather waterproof sheet,
a fixed region between the topsheet fold-over portion and the gather waterproof sheet extending from a position located within 5 mm from an end of the topsheet fold-over portion on a side of the second gather elastic member toward the starting end, to at least the starting end.

### (Operational effect)

As described in the aspect above, the topsheet is extended sideways, folded back along the main body of the standing gather, and fixed on the standing portion. The restoring force generated by the fold-over portion of the topsheet acts to raise the third portion from the starting end. The topsheet also enhances the stiffness of the third portion, so that bending is even less likely to occur between the second gather elastic member and the starting end.

### <Third aspect>

The disposable wearing article according to the second aspect, further including a back waterproof sheet made of a waterproof resin film provided on a back side of the absorbent,
wherein the back waterproof sheet includes a back waterproof sheet extension extending out to either side in the width direction of the absorbent,
the back waterproof sheet extension including a back waterproof sheet fold-over portion folded back at the starting end onto the front side,
the back waterproof sheet fold-over portion constituting the gather waterproof sheet.

### (Operational effect)

As described in the aspect above, the back waterproof sheet made of a waterproof resin film is extended sideways, folded back along the main body of the standing gather, and fixed on the standing portion. This is preferable because the restoring force generated by the fold-over portion of the back waterproof sheet acts to raise the third portion from the starting end.

### <Fourth aspect>

The disposable wearing article according to the third aspect, further including an outer nonwoven fabric provided on a back side of the absorbent,
wherein the outer nonwoven fabric includes an outer nonwoven extension extending out to either side in the width direction of the absorbent,
the outer nonwoven extension including an outer fold-over portion folded back at the starting end onto the front side,
the outer nonwoven fold-over portion extending from the starting end to a position located within ± 5 mm in the width direction relative to the end of the gather waterproof sheet on the side of the second gather elastic member, and being fixed to the gather waterproof sheet,
a fixed region between the outer nonwoven fold-over portion and the gather waterproof sheet extending from a position located within 5 mm from an end of the outer nonwoven fold-over portion on a side of the second gather elastic member toward the starting end, to at least the starting end.

### (Operational effect)

As described in the aspect above, the outer nonwoven fabric is extended sideways, folded back along the main body of the standing gather, and fixed on the standing portion. The restoring force generated by the fold-over portion of the outer nonwoven fabric acts to raise the third portion from the starting end. The nonwoven fabric also enhances the stiffness of the third portion, so that bending is even less likely to occur between the second gather elastic member and the starting end.

### <Fifth aspect>

The disposable wearing article according to any one of the first to fourth aspects, wherein the third portion has a 80 to 150 mm bending resistance as determined by a method known as 45° cantilever method.

### (Operational effect)

From the perspective of achieving the above-mentioned advantageous effects, the stiffness of the third portion may be within the range specified according to this aspect, without limitation.

### <Sixth aspect>

The disposable wearing article according to any one of second to fifth aspects, wherein the absorbent includes a narrower part with a reduced width in a middle part in the front-back direction,
the narrower part in a portion with a smallest width having a side edge distanced by 5 to 20 mm in the width direction from the starting end,
the starting end being distanced in the width direction from the second gather elastic member by 10 to 25 mm.

### (Operational effect)

It is the common practice to provide a narrower part in the middle part in the front-back direction of the absorbent where the width is reduced from that of the front and rear parts for better fit in the crotch portion. In this case, the areas between the side edges of the narrower part and the starting ends of the gathers increase the height of the standing gathers in the crotch portion. This increased height makes the forces applied to the third portion more complex, thereby making unwanted bending more likely to occur between the second gather elastic member and the starting end. Therefore, the gather waterproof sheet enhancing the stiffness of the third portion has particular significance in the above-described aspect.

### Advantageous Effects of Invention

The present invention provides advantages including improved fit in fold-over type standing gathers.

### Brief Description of Drawings

[Fig. 1]

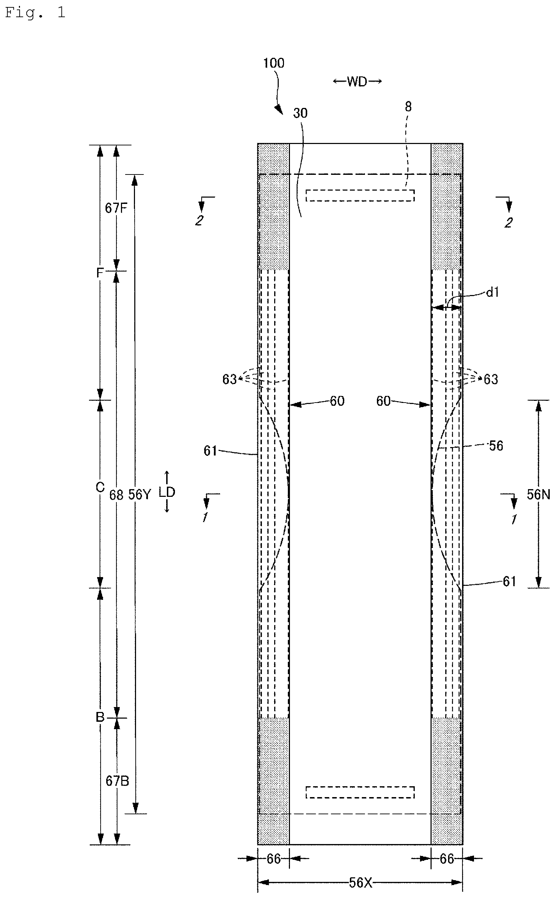

   Fig. 1 is a plan view showing an inner surface of a pad-type disposable diaper.
[Fig. 2]

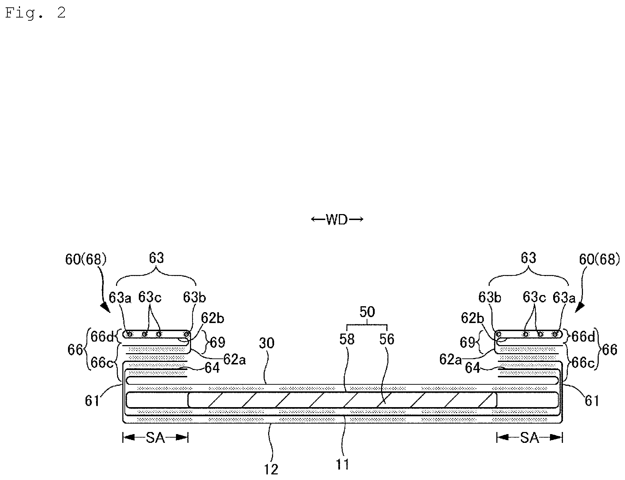

   Fig. 2 is a sectional view taken along 1-1 in Fig. 1.
[Fig. 3]

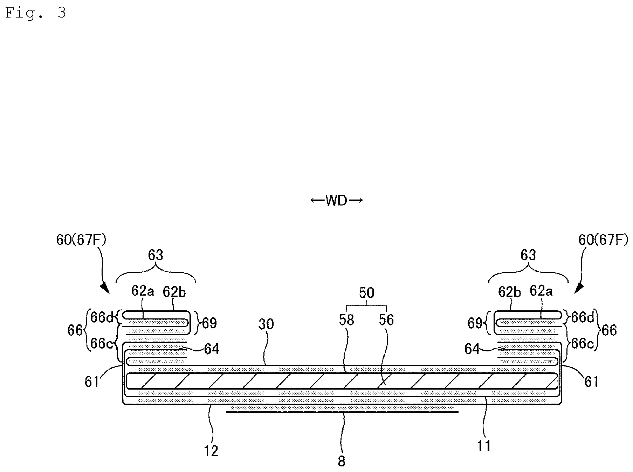

   Fig. 3 is a sectional view taken along 2-2 in Fig. 1.
[Fig. 4]

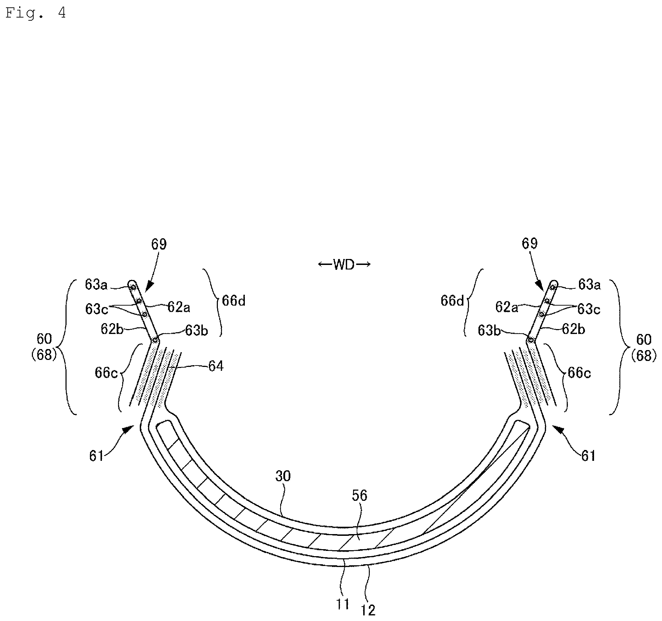

   Fig. 4 is a sectional view taken along 1-1 in Fig. 1 of a pad-type disposable diaper when worn.
[Fig. 5]

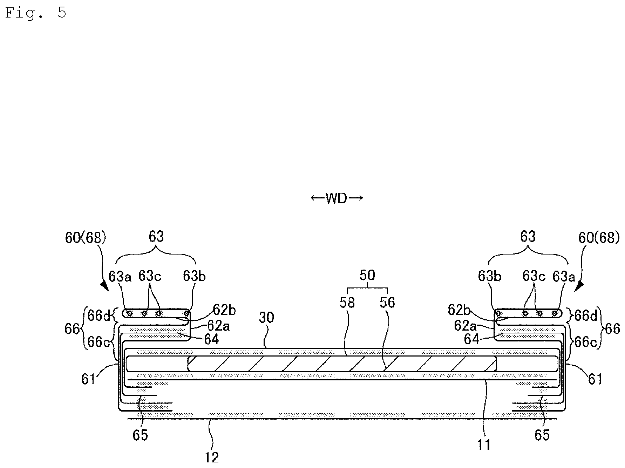

   Fig. 5 is a sectional view taken along 1-1 in Fig. 1 of a pad-type disposable diaper in another embodiment.
[Fig. 6]

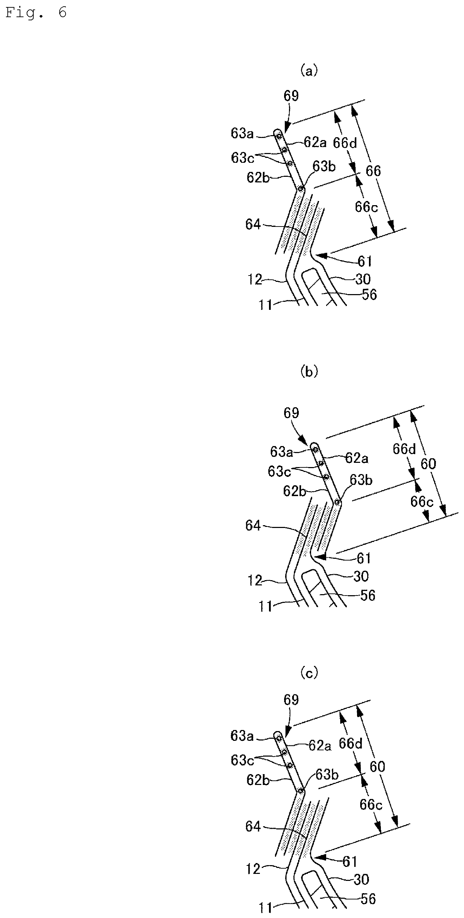

   Fig. 6 is an enlarged view of a cross section of essential portions of standing gathers.
[Fig. 7]

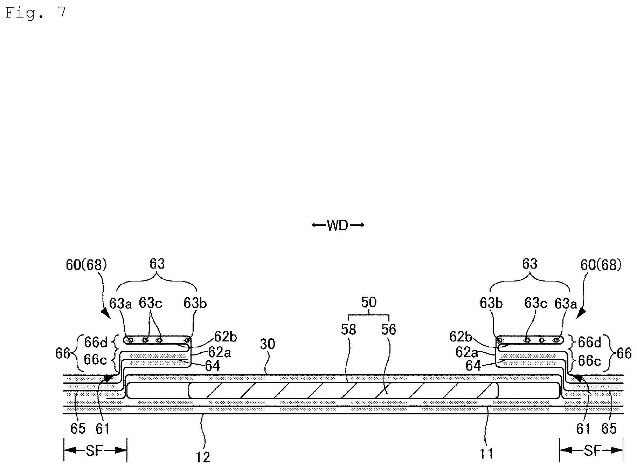

   Fig. 7 is a sectional view taken along 1-1 in Fig. 1 of a pad-type disposable diaper in another embodiment.

### Description of Embodiments

Hereinafter, an example of a pad-type disposable diaper will be described in detail with reference to the accompanying drawings. In fixed or joined portions other than those described below, each component adjacent to each other in a thickness direction is fixed or joined as necessary in the same manner as in known diapers. Portions with dotted patterns in sectional views indicate an adhesive such as a hot-melt adhesive as means for such fixing or joining. Hot-melt adhesives can be applied by known methods such as slot application, continuous linear or dotted bead application, spray application in a spiral shape, a Z-shape, a wavy shape, or the like, or pattern coating (transfer of hot-melt adhesive by letterpress method). In place of or in addition to the above, in a fixing portion of an elastic member, a hot-melt adhesive may be applied to an outer peripheral surface of the elastic member and the elastic member may be fixed to an adjacent member. Any of hot-melt adhesives, such as EVA-based, adhesive rubber-based (elastomer-based), polyolefin-based, and polyester/polyamide-based hot-melt adhesives, can be used without any particular limitations. As the fixing or joining means for joining each component, means that involve material welding such as heat sealing or ultrasonic sealing can be used. In portions where liquid permeability in a thickness direction is required, adjacent components in the thickness direction are fixed or joined in an intermittent pattern. For example, when such intermittent fixing or joining is to be performed using a hot-melt adhesive, intermittent pattern application in a spiral shape, a Z-shape, or a wavy shape can be suitably used, and when applying to an area that is equal to or larger than an application width by a single nozzle, intermittent pattern application in a spiral shape, a Z-shape, or a wavy shape can be performed with or without spacing in a width direction WD. As the joining means for joining each component, means that involve material welding such as heat sealing or ultrasonic sealing can also be used.

In the following description, any known nonwoven fabric may be appropriately used depending on the location and intended function. The constituent fibers of the nonwoven fabric may be selected without particular limitation from synthetic fibers such as polyolefin fibers (e.g., polyethylene or polypropylene), polyester fibers, and polyamide fibers (including composite fibers such as core-sheath fibers as well as single-component fibers); regenerated fibers such as rayon or cuprammonium rayon; and natural fibers such as cotton. These fibers may also be used in combination. To increase the flexibility of the nonwoven fabric, it is preferable that the constituent fibers be crimped fibers. The constituent fibers of the nonwoven fabric may be hydrophilic fibers (including fibers rendered hydrophilic by a hydrophilizing agent), hydrophobic fibers, or water-repellent fibers (including fibers rendered water-repellent by a water-repellent agent). Nonwoven fabrics are generally classified according to fiber length, sheet-forming method, fiber-bonding method, or lamination structure into short-fiber nonwoven fabrics, longfiber nonwoven fabrics, spunbond nonwoven fabrics, meltblown nonwoven fabrics, spunlace nonwoven fabrics, thermal-bonded (air-through) nonwoven fabrics, needle-punched nonwoven fabrics, point-bonded nonwoven fabrics, and laminated nonwoven fabrics (including SMS nonwoven fabrics, SMMS nonwoven fabrics, and the like, in which a meltblown layer is interposed between spunbond layers). Any of these nonwoven fabrics may be used.

Figs. 1 to 4 illustrate an example of a pad-type disposable diaper 100. This pad-type disposable diaper 100 is used as a liner laid on the inner surface of an outer cover such as a pull-up disposable diaper, so that it is narrow and has no side flaps SF extending sideways from the absorbent 56. Optionally, the diaper may include a pair of side flaps SF without the absorbent 56 extending out from both side edges of the absorbent 56 as shown in Fig. 7. The pad-type disposable diaper 100 in this example includes a crotch portion M, a front region F extending forward from the crotch portion M, and a rear region B extending rearward from the crotch portion M. Note that, when the absorbent 56 includes a narrower part 56N to be described later, the "crotch portion M" refers to an area in the front-back direction LD including this narrower part 56N. When the absorbent 56 does not have the narrower part 56N, the "crotch portion M" refers to a portion positioned in the center in the front-back direction LD and a portion spanning 20 to 30% of the total product length Y in the front-back direction LD.

While the pad-type disposable diaper 100 in this example is rectangular with the long sides (side edges) extending along the front-back direction, suitable modifications are possible. For example, the four corners could be cut off, or both sides of the crotch portion M could be cut off.

The pad-type disposable diaper 100 in this example includes, as shown in Figs. 1 to 4, an absorbent 56 for absorbing and retaining urine, a wrapping sheet 58 wrapping the absorbent 56, a topsheet 30 provided on the front side of the absorbent 56, a back waterproof sheet 11 provided on the back side of the absorbent 56, and standing gathers 60 provided on both sides in the width direction WD of the front surface, which stand up from starting ends 61 that extend along the front-back direction LD.

### (Topsheet)

The topsheet 30 has liquid permeability and can be, for example, a perforated or non-perforated nonwoven fabric, or a perforated plastic sheet. The topsheet 30 may be a single sheet or a laminated sheet obtained by bonding together two or more sheets. Likewise, the topsheet 30 may be a single sheet or two or more sheets with respect to its planar direction.

As shown in the illustrated example, the topsheet 30 includes side portions extending such as to form part of the standing gathers 60. Optionally, as shown in the example in Fig. 5, the side portions may be folded back over to the back side at the side edges of the absorption element 50 and passed through between the back waterproof sheet 11 and the standing gathers 60 over to the back side of the absorption element 50. Alternatively, as shown in the example in Fig. 7, the side portions may be extended beyond the side edges of the absorption element 50 rather than folded back there, to form part of the side flaps SF.

For the purpose of preventing misalignment relative to a member on the back side, the topsheet 30 is desirably fixed to the adjacent member on the back side by joining means using material welding such as heat sealing or ultrasonic sealing, or by a hot-melt adhesive. In the illustrated example, the topsheet 30 is fixed to a portion of the wrapping sheet 58 positioned on the front side of the absorbent 56 with the hot-melt adhesive applied to the back side of the topsheet 30.

### (Back waterproof sheet)

Examples of the material for the back waterproof sheet 11 include, but not particularly limited to, waterproof resin films made of polyolefin-based resins such as polyethylene or polypropylene, and waterproof nonwoven fabrics. From the perspective of reducing stuffiness, a moisture-permeable material is preferably used for the back waterproof sheet 11. A preferable example of a moisture-permeable, waterproof resin film is a microporous waterproof resin film obtained by uniaxially or biaxially stretching a formed sheet of polyolefin-based resin such as polyethylene and polypropylene kneaded with inorganic fillers.

### (Absorbent)

The absorbent 56 can be formed of a fiber aggregate. Applicable fiber aggregates include accumulated products of staple fibers such as cotton pulp or synthetic fibers, and filament aggregates obtained by spreading, as required, tows (fiber bundles) of synthetic fibers such as cellulose acetate. In the case with the layer of cotton pulp or staple fiber, it may have a basis weight of about 100 to 450 g/m², for example, and in the case with the filament aggregate, it may have a basis weight of about 30 to 120 g/m², for example. When using synthetic fiber, it should preferably have a thickness of 1 to 16 dtex, for example, and more preferably 1 to 10 dtex, and even more preferably 1 to 5 dtex. When using a filament aggregate, it should preferably be made of crimped fiber, while it can consist of non-crimped fiber. When using crimped fiber, it may have 5 to 75 crimps per 2.54 cm, for example, preferably 10 to 50, and more preferably 15 to 50. Fibers with even crimp may also be used. It is preferable to disperse and hold superabsorbent polymer particles in the absorbent 56.

The absorbent 56 can have any size determined as appropriate as long as it extends from front to back of the crotch portion M. Preferably, the absorbent 56 extends to peripheral edges or near the edges of the product in the front-back direction LD and the width direction WD. Reference signs 56X and 56Y respectively denote the full width and the full length of the absorbent 56.

While the absorbent 56 can be rectangular, it may have a front region F, a rear region B, and a narrower part 56N between them where the width is reduced from that of the front region F and the rear region B (substantially an hourglass shape in a plan view) as shown in Fig. 1. This shape is preferable as it offers better fit of the absorbent 56 itself and the standing gathers 60 around the legs. In this case, the width of the narrower part 56N (width of the narrowest portion of the absorbent 56) may be determined as appropriate. For example, it could be 0.5 to 0.8 times the full width 56X of the absorbent 56.

### (Superabsorbent polymer particle)

The absorbent 56 can partly or entirely contain superabsorbent polymer particles. The superabsorbent polymer particles include not only "particles" but also "powder." The superabsorbent polymer particles commonly used in such disposable diapers can be used as they are. For example, it is preferable to use particles in which the proportion of particles that remain on a 500 µm standard sieve (JIS Z8801-1: 2006) after sieving (shaking for 5 minutes) is 30% by weight or less. Furthermore, it is preferable to use particles in which the proportion of particles that remain on a 180 µm standard sieve (JIS Z8801-1: 2006) after sieving (shaking for 5 minutes) is 60% by weight or more.

The superabsorbent polymer particles, while not limited to a specific type, are preferably made of a material with a water absorption capacity of 40 g/g or more. Examples of the superabsorbent polymer particles include starch-based particles, cellulose-based particles, and synthetic polymerbased particles. Starch-acrylic acid (salt) graft copolymers, saponified products of starch-acrylonitrile copolymers, crosslinked products of sodium carboxymethyl cellulose, and acrylic acid (salt) polymers, and the like can be used. While the common powdery or particulate forms are the preferable shapes of the superabsorbent polymer particles, other shapes can also be used.

It is preferable to use superabsorbent polymer particles with a water absorption rate of 70 seconds or less and particularly those with a water absorption rate of 40 seconds or less. If the water absorption rate is too low, a phenomenon known as returning, in which liquid absorbed into the absorbent 56 flows back out of the absorbent 56, is likely to occur.

Furthermore, as the superabsorbent polymer particles, it is preferable to use those having a gel strength of 1000 Pa or more. As a result, even when the absorbent 56 is bulky, the tacky feeling after liquid absorption can be effectively suppressed.

The basis weight of the superabsorbent polymer particles can be appropriately determined according to the required absorption capacity for the absorbent 56. Accordingly, although the basis weight cannot be uniformly specified, it can be in the range of 50 to 350 g/m². When the basis weight of the polymer is less than 50 g/m², it becomes difficult to achieve the absorption capacity. When the basis weight exceeds 350 g/m², the effect reaches saturation.

The ratio of the fibers to the superabsorbent polymer particles in the absorbent 56 is not particularly limited. For example, the weight ratio of fibers to superabsorbent polymer particles may range from 40:60 to 65:35.

### (Wrapping sheet)

To ensure or enhance the shape retainability of the absorbent 56, it is preferable to wrap the entire absorbent 56 with a wrapping sheet 58. Alternatively, the wrapping sheet 58 may be omitted. Materials that can be used as the wrapping sheet 58 include tissue paper, particularly crepe paper, nonwoven fabric, poly-laminated nonwoven fabric, and any perforated sheets. If the absorbent 56 contains superabsorbent polymer particles, it is desirable that the sheet does not allow the polymer particles to pass through. When using nonwoven fabric instead of crepe paper, it is particularly preferable to use a hydrophilic SMS nonwoven fabric (such as SMS and SSMMS). Polypropylene, or polyethylene/polypropylene composites can be used as the material. The wrapping sheet 58 should preferably have a basis weight of 5 to 40 g/m² and more preferably 10 to 30 g/m².

The wrapping form of the wrapping sheet 58 can be appropriately determined. From the viewpoints of ease of manufacture and prevention of leakage of superabsorbent polymer particles from the front and rear edges, it is preferable that the wrapping sheet 58 be wound in a cylindrical manner so as to surround the front and back surfaces and both side surfaces of the absorbent 56, with front and rear edge portions extending beyond the front and rear of the absorbent 56, and that overlapping portions of the wound sheet and overlapping portions of the extended front and rear edge portions be joined by joining means such as a hot-melt adhesive, material welding, or the like.

### (Outer nonwoven fabric)

In cases where it is undesirable for the back waterproof sheet 11 to be exposed on the back side, such as when the back waterproof sheet 11 is a waterproof resin film, an outer nonwoven fabric 12 may be provided on the back side of the product to cover the back waterproof sheet 11, as illustrated. Alternatively, the outer nonwoven fabric 12 can be omitted. A bonding member 8 may or may not be provided as required on the outer side of the outer nonwoven fabric 12 to allow secure attachment or tacking to an outer diaper or underwear, similarly to known pad-type disposable diapers.

### (Standing gather)

The standing gathers 60 are provided on both sides in the width direction WD of the front surface, such as to stand up from the starting ends 61 that extend along the front-back direction LD. The standing gather 60 should preferably extend from the front region F to the rear region B, particularly from a point farther forward than the front end of the absorbent 56 to a point farther back than the rear end of the absorbent 56.

More specifically, the standing gather 60 shown in Fig. 2 to Fig. 7 includes: a main body 66 extending toward the center in the width direction WD from the starting end 61 that extends along the front-back direction LD; a front fallen section 67F formed by fixing a front end portion of the main body 66 in a fallen state; a rear fallen section 67B formed by fixing a rear end portion of the main body 66in a fallen state; a non-fixed standing portion 68 located between the front fallen section 67F and the rear fallen section 67B of the main body 66; and a plurality of gather elastic members 63 extending along the front-back direction LD and spaced apart in the width direction WD in the standing portion 68. The gather elastic members 63 are fixed to the standing portion 68 along the front-back direction LD in a stretched state. As a result, the standing portion 68 shrinks from its natural length and stands up with the gather elastic members 63, and stretches with the gather elastic members 63 according to an external force, conforming to (fit) a body surface.

The standing gather 60 in the illustrated example is a folded-back type. The front fallen section 67F and the rear fallen section 67B each include a first portion 66a extending from the starting end 61 toward the center in the width direction WD, and a second portion 66b extending from the distal end of this first portion 66a outwardly in the width direction WD. The standing portion 68 includes a third portion 66c continuous from the first portion 66a of the front fallen section 67F to the first portion 66a of the rear fallen section 67B and a fourth portion 66d continuous from the second portion 66b of the front fallen section 67F and the second portion 66b of the rear fallen section 67B.

The characteristic feature of the gather elastic member 63 is that it includes a first gather elastic member 63a attached to the distal end of the fourth portion 66d opposite to the third portion 66c, and a second gather elastic member 63b attached along the boundary between the third portion 66c and the fourth portion 66d, and that the gather elastic member 63 is not present in a region from the second gather elastic member 63b toward the starting end 61. As a result, the fourth portion 66d stands up at a low angle and makes surface contact with the body surface, and the third portion 66c rises up higher. The fourth portion 66d may include one or a plurality of third gather elastic members 63c spaced apart in the width direction WD between the first gather elastic member 63a and the second gather elastic member 63b.

A gather waterproof sheet 64 made of a waterproof resin film and covering the third portions 66c is fixedly attached to the main body 66 over the entire length in the front-back direction LD. The gather waterproof sheet 64 extends from a position located within 5 mm (more preferably, within 3 mm) in the width direction WD from the second gather elastic member 63b toward the starting end 61, to at least the starting end 61. A fixed region between the gather waterproof sheet 64 and the main body 66 extends from a position located within 5 mm (more preferably within 3 mm) in the width direction WD from an end of the gather waterproof sheet 64 on the side of the second gather elastic member 63b toward the starting end 61, to at least the starting end 61. Further provided is a gather nonwoven fabric 62 covering the third portion 66c and the fourth portion 66d. The fourth portion 66d is free of the gather waterproof sheet 64 and has fewer sheet layers than the third portion 66c. More specifically, in the example shown in Fig. 2 to Fig. 4, the third portion 66c has five sheet layers (two layers of gather nonwoven fabric 62, one layer each of the outer nonwoven fabric 12, gather waterproof sheet 64, and topsheet 30). The fourth portion 66d has two sheet layers (only two layers of gather nonwoven fabric 62). Accordingly, in the standing gather 60 of this example, substantially the entire third portion 66c of the third and fourth portions 66c and 66d constitutes a high-stiffness region to which a waterproof resin film having particularly high stiffness (the gather waterproof sheet 64) is bonded. Since a region near the distal end of the third portion 66c is lifted by the second gather elastic member 63b, a fold-back of the standing portion 68 is unlikely to occur at locations other than along the second gather elastic member 63b, and bending is particularly unlikely to occur between the second gather elastic member 63b and the starting end 61. Therefore, the third portion 66c not only ensures a certain stand-up height, but also provides shape retainability, in addition to high waterproof properties given by the gather waterproof sheet 64. On the other hand, the fourth portion 66d is free of the waterproof resin film (gather waterproof sheet 64) with high stiffness, and has fewer sheet layers than the third portion 66c, so that it feels softer (less stiff) when it contacts the skin. Unlike the illustrated example, the gather waterproof sheet 64 may cover only the standing portion 68 over the entire length in the front-back direction LD. In this respect, rather than being sandwiched between layers of the gather nonwoven fabric 62 as in the example shown in Fig. 5, the gather waterproof sheet 64 may be joined more inside than the gather nonwoven fabric 62 (closer to the center in the width direction WD) and not sandwiched between layers of the gather nonwoven fabric 62 as shown in Figs. 2 and 3. This is preferable because the second portion 66b and the fourth portion 66d are more readily folded back onto the first portion 66a and the third portion 66c, respectively.

In the illustrated example, the first portion 66a and the third portion 66c include at least one layer of gather nonwoven fabric 62 in addition to the gather waterproof sheet 64. The second portion 66b and the fourth portion 66d include at least one layer of gather nonwoven fabric 62. The gather nonwoven fabric 62 may either be a dedicated nonwoven fabric, or an extended portion of another sheet near the starting end 61. In the illustrated example, the standing gather 60 is formed by a gather assembly 69 having the entire length and width covering the first portion 66a to the fourth portion 66d. The gather assembly 69 is made up of: a gather nonwoven fabric 62, which includes an inner layer 62a extending from the starting end 61 to the distal ends of the second portion 66b and the fourth portion 66d, and an outer layer 62b extending from the distal ends of the second portion 66b and the fourth portion 66d, where it is folded back outwardly, to the starting end 61; and a gather elastic member 63 sandwiched between the inner layer 62a and the outer layer 62b in the fourth portion 66d. The gather assembly 69 may be bonded to an opposite member at a base portion 65 that is extended via the starting end 61 of the standing portion over to the back side of the absorbent 56, as in the example shown in Fig. 5. Alternatively, as shown in Figs. 2 and 3, in the case where there are components forming part of the main body (e.g., topsheet 30, back waterproof sheet 11, and outer nonwoven fabric 12 in the illustrated example), the components extending from a position located on the central side in the width direction WD relative to the starting end 61, through the starting end 61, and into the main body 66 (at least the first portion 66a and the third portion 66c), the gather assembly may be bonded to the portions of these components that form the main body (folded-back portions of the topsheet 30, back waterproof sheet 11, and outer nonwoven fabric 12 in the illustrated example). In the latter case, the side edges of the portions of the components forming the main body may be positioned within 5 mm (more preferably, within 3 mm) in the width direction WD from the second gather elastic member 63b toward the starting end 61, and the gather assembly 69 may be bonded to the outer side of the portions of the components forming the main body, as in the example shown in Figs. 2 to 4, 6(a), and 6(c). This is preferable because the second portion 66b and the fourth portion 66d are more readily folded back onto the first portion 66a and the third portion 66c, respectively. Alternatively, the gather assembly 69 may be bonded to the inner side of the portions of the components forming the main body as shown in Fig. 6(b).

The topsheet 30 may be folded back over to the back side of the absorbent 56 along the side edges of the absorbent 56 as in the example shown in Fig. 5. Alternatively, as shown in Figs. 2 and 3, the topsheet 30 may preferably include a topsheet extension extending out to either side in the width direction WD of the absorbent 56, the topsheet extension including a topsheet fold-over portion folded back at the starting end 61 onto the front side. Preferably, the topsheet fold-over portion extends from the starting end 61 to a position located within ± 5 mm (more preferably, within ± 3 mm) in the width direction WD from the end of the gather waterproof sheet 64 on the side facing the second gather elastic member 63b, and is fixed to the gather waterproof sheet 64. Preferably, a fixed region between the topsheet fold-over portion and the gather waterproof sheet 64 extends from a position located within 5 mm (more preferably, within ± 3 mm) in the width direction WD from the end of the topsheet fold-over portion on the side facing the second gather elastic member 63b toward the starting end 61, to at least the starting end 61. Namely, it is preferable to form at least the third portions 66c (as well as the first portions 66a in the illustrated example) of the standing gathers 60 with both side portions of the topsheet 30. As described above, the topsheet 30 is extended sideways, folded back along the main body of the standing gather 60, and fixed on the standing portion 68. As a result, the restoring force generated by the fold-over portion of the topsheet 30 acts to raise the third portion 66c from the starting end 61, and the stiffness of the third portion 66c is further enhanced, so that bending is even less likely to occur between the second gather elastic member 63b and the starting end 61. The fold-over portion of the topsheet should preferably form the innermost surface of the main body 66, and cover the entire, or substantially entire, inner surface of the gather waterproof sheet 64 as in the illustrated example to prevent the waterproof resin film from touching the skin.

The back waterproof sheet 11 may be disposed only on the back side of the absorbent 56 as in the example shown in Fig. 5, in which case the gather waterproof sheet 64 is a separate waterproof resin film from the back waterproof sheet 11. In the case where the back waterproof sheet 11 is made of a waterproof resin film, the back waterproof sheet 11 may include a back waterproof sheet extension extending out to either side in the width direction of the absorbent 56 as shown in Figs. 2 and 3. The back waterproof sheet extension may include a back waterproof sheet fold-over portion folded back at the starting end 61 onto the front side, this back waterproof sheet fold-over portion constituting the gather waterproof sheet 64. Namely, it is preferable to form at least the third portions 66c (as well as the first portions 66a in the illustrated example) of the standing gathers 60 with both side portions of the back waterproof sheet 11. As a result, the number of waterproof resin films used can be reduced and the restoring force generated by the fold-over portion of the back waterproof sheet 11 acts to raise the third portion 66c from the starting end 61.

In the case where the outer nonwoven fabric 12 is provided, it is preferable that the outer nonwoven fabric 12 include an outer extension extending out to either side in the width direction WD of the absorbent 56, the outer extensions each including an outer fold-over portion folded back at the starting end 61 onto the front side. Preferably, the outer fold-over portion extends from the starting end 61 to a position located within ± 5 mm (more preferably, within ± 3 mm) in the width direction WD from the end of the gather waterproof sheet 64 on the side facing the second gather elastic member 63b, and is fixed to the gather waterproof sheet 64. Preferably, a fixed region between the outer fold-over portion and the gather waterproof sheet 64 extends from a position located within 5 mm (more preferably, within 3 mm) in the width direction WD from the end of the outer extension on the side facing the second gather elastic member 63b toward the starting end 61, to at least the starting end 61. Namely, it is preferable to form at least the third portions 66c (as well as the first portions 66a in the illustrated example) of the standing gathers 60 with both side portions of the outer nonwoven fabric 12. As described above, the outer nonwoven fabric 12 is extended sideways, folded back along the main body of the standing gather 60, and fixed on the standing portion 68. As a result, the restoring force generated by the fold-over portion of the outer nonwoven fabric 12 acts to raise the third portion 66c from the starting end 61. The outer nonwoven fabric 12 also enhances the stiffness of the third portion 66c, so that bending is even less likely to occur between the second gather elastic member 63b and the starting end 61. As shown in the illustrated example, the fold-over portion of the outer nonwoven fabric should preferably form the outermost surface of the main body 66, and cover the entire or substantially entire, outer surface of the gather waterproof sheet 64 to prevent the waterproof resin film from touching the skin.

The gather waterproof sheet 64, and the sheets forming the third portion 66c other than the gather assembly 69 (topsheet 30 and outer nonwoven fabric 12 in the illustrated example), may have their distal ends facing the second gather elastic member 63b aligned (stacked in the thickness direction) as shown in Fig. 2 to Fig. 4, or displaced from each other. For example, the sheets forming the third portion 66c other than the gather assembly 69 and the gather waterproof sheet 64 may have their distal ends arranged such that a distance in the width direction WD between each distal end and the second gather elastic member 63b increases toward inner sheets (i.e., in the order of the outer nonwoven fabric 12, gather waterproof sheet 64, topsheet 30 in the illustrated example), with each increase being within 5 mm (more preferably, not more than 3 mm), as shown in Fig. 6(a). This is preferable because a thickness change of the third portion 66c becomes smoother, thereby providing a favorable feel on the skin.

It is also preferable that the gather waterproof sheet 64 be entirely concealed as shown in Fig. 6(b) by arranging a distance in the width direction WD between a distal end of the gather waterproof sheet 64 and the second gather elastic member 63b to be greater by no more than 5 mm (more preferably, no more than 3 mm) than a distance in the width direction WD between distal ends of sheets forming the third portion 66c other than the gather assembly 69 and the second gather elastic member 63b. From the same perspective, it is also preferable that the gather waterproof sheet 64 be entirely concealed by arranging the distal ends of the sheets forming the third portion 66c other than the gather assembly 69 and the gather waterproof sheet 64 such that a distance in the width direction WD between each distal end and the second gather elastic member 63b increases toward outer sheets (i.e., in the order of the topsheet 30, gather waterproof sheet 64, and outer nonwoven fabric 12 in the illustrated example), with each increase being within 5 mm (more preferably, not more than 3 mm).

The bending resistance of the third portion 66c, measured by cutting out the entire third portion 66c and using the 45° cantilever method, is preferably 80 to 150 mm. The bending resistance of each sheet constituting the third portion 66c may be appropriately selected. Preferably, the topsheet 30 has a bending resistance of 10 to 80 mm (more preferably 20 to 70 mm) according to the 45° cantilever method. The outer nonwoven fabric 12 preferably has a bending resistance of 10 to 80 mm (more preferably 20 to 70 mm) according to the 45° cantilever method. The gather nonwoven fabric 62 also preferably has a bending resistance of 10 to 80 mm (more preferably 20 to 70 mm) according to the 45° cantilever method. Further, because the fourth portion 66d does not include the topsheet 30, the gather waterproof sheet 64, or the backsheet, its bending resistance is preferably the same as, or close to, the bending resistance of the third portion 66c with the topsheet 30, the gather waterproof sheet 64, and the backsheet removed (i.e., 0.2 to 0.8 times, more preferably 0.3 to 0.7 times, the bending resistance of the third portion 66c).

It is the common practice to provide the narrower part 56N in the middle in the front-back direction LD of the absorbent 56 where the width is reduced from that of the front and rear parts as shown in Fig. 1 for better fit in the crotch portion M. In this case, the areas SA (see Fig. 2) between the side edges of the narrower part 56N and the starting ends 61 of the standing gathers 60 increase the stand-up height of the standing gathers 60 in the crotch portion M. For example, if this area SA measures about 8 to 25 mm in the width direction WD and the starting end 61 is spaced from the second gather elastic member 63b in the width direction WD by a distance d1 (see Fig. 1) of about 10 to 25 mm, the increased stand-up height due to the area SA makes the forces applied to the third portion 66c more complex, thereby making unwanted bending more likely to occur between the second gather elastic member 63b and the starting end 61. Therefore, the gather waterproof sheet 64 enhancing the stiffness of the third portion 66c as described above has particular significance in such cases.

### <Explanation of terms in specification>

The following terms in the specification have the following meanings unless otherwise described in the specification.
- The "front-back direction" means the direction denoted by reference sign LD in the drawings (vertical direction), the "width direction" means the direction denoted by WD in the drawings (left-right direction). The front-back direction and the width direction are orthogonal to each other.
- The "front side" means a side closer to the skin of the wearer when being worn, and the "back side" means a side farther from the skin of the wearer when being worn.
- The "front surface" means a surface closer to the skin of the wearer when being worn, and the "back surface" means a surface farther from the skin of the wearer when being worn.
- The "stretch rate" means a value when a natural length is considered 100%. For example, a stretch rate of 200% is synonymous with an elongation factor of 2.
- The "gel strength" is measured in the following manner. 1.0 g of superabsorbent polymer is added to 49.0 g of artificial urine (a mixture of 2 wt% urea, 0.8 wt% sodium chloride, 0.03 wt% calcium chloride dihydrate, 0.08 wt% magnesium sulfate heptahydrate, and 97.09 wt% ion-exchanged water), and the mixture is stirred using a stirrer. The generated gel is left to stand for three hours in a constant temperature and humidity chamber at 40°C × 60% RH, and then returned to room temperature. The gel strength is measured using a curdmeter (Curdmeter-MAX ME-500 manufactured by I. techno Engineering Co., Ltd).
- The "basis weight" is measured as follows. After pre-drying the specimen or the test piece, the specimen or the test piece is left in the test room or apparatus under standard conditions (test site is at temperature 23 ± 1°C, relative humidity 50 ± 2%) to reach a constant volume. Pre-drying refers to bringing the specimen or the test piece to a constant volume at a temperature of 100°C. Note that fibers with an official regain of 0.0% need not be pre-dried. From the test piece having reached a constant volume, a specimen with dimensions of 100 mm × 100 mm is cut out using a sampling mold plate (100 mm × 100 mm). The weight of the specimen is measured and multiplied by 100 to calculate the weight per square meter, which is adopted as the basis weight.
- The "thickness" of thick members, such as the absorbent 56, the absorption element 50, and the inner member 200, is measured using a thickness measurement device manufactured by OZAKI MFG. CO., Ltd. (PEACOCK, dial thickness gauge, Type H (with a measurement range of 0 to 10 mm, a circular terminal having a measurement area diameter of 10 mm, a measuring force of approximately 1.7 N, and a pressure of approximately 21.7 KPa)). The measurement is performed with a sample and the thickness measurement device put in a horizontal position.
- The "thickness" of thin sheets, such as nonwoven fabric, the "compression stiffness LC in the thickness direction", and the "resilience RC in the thickness direction" are automatically measured using an automatic thickness measurement device (KES-G5, handy compression measurement program) under conditions of a load of 0.098 N/cm² and a pressed area of 2 cm².
- The water absorption capacity is measured in accordance with JIS K7223-1996, "Testing Method for Water Absorption Capacity of Superabsorbent Polymers."
- The water absorption rate is defined as the "time until the endpoint" when the test in JIS K7224-1996, "Testing Method for Water Absorption Rate of Superabsorbent Polymers," is conducted using 2 g of superabsorbent polymer and 50 g of physiological saline.
- The "unfolded state" means a state in which the article is laid flat without contraction (including any contraction caused by elastic members) or slack.
- Unless otherwise specified, the dimensions of each portion refer to the dimensions in the unfolded state and not in a natural length state.
- If there is no description of environmental conditions, a test or a measurement is to be performed in a laboratory or an apparatus under standard conditions (the test site shall be at a temperature of 23 ± 1°C and a relative humidity of 50 ± 2%).

### Industrial Applicability

The present invention is suitable for disposable pull-up diapers such as the example described above.

### Reference Signs List

- 11: Back waterproof sheet
- 30: Topsheet
- 56: Absorbent
- 56N: Narrower part
- 58: Wrapping sheet
- 60: Standing gather
- 61: Starting end
- 62: Gather nonwoven fabric
- 62a: Inner layer
- 62b: Outer layer
- 63: Gather elastic member
- 63a: First gather elastic member
- 63b: Second gather elastic member
- 63c: Third gather elastic member
- 64: Gather waterproof sheet
- 65: Base portion
- 66: Main body
- 66a: First portion
- 66b: Second portion
- 66c: Third portion
- 66d: Fourth portion
- 67F: Front fallen section
- 67B: Rear fallen section
- 68: Standing portion
- 69: Gather assembly
- B: Rear region
- F: Front region
- LD: Front-back direction
- M: Crotch portion
- WD: Width direction

## Claims

1. A disposable wearing article with a standing gather provided on either side in a width direction of a surface to stand up from a starting end thereof that extends along a front-back direction,
the standing gather comprising: a main body extending from the starting end toward a central region in the width direction; a front fallen section formed by fixing a front end portion of the main body in a fallen state; a rear fallen section formed by fixing a rear end portion of the main body in a fallen state; a non-fixed standing portion located between the front fallen section and the rear fallen section of the main body; and a plurality of gather elastic members extending along the front-back direction and spaced apart in the width direction in the standing portion,
the front fallen section and the rear fallen section each including a first portion extending from the starting end toward the central region in the width direction and a second portion extending from a distal end of this first portion outwardly in the width direction, the standing portion including a third portion continuous from the first portion of the front fallen section to the first portion of the rear fallen section and a fourth portion continuous from the second portion of the front fallen section and the second portion of the rear fallen section,
the gather elastic members including a first gather elastic member attached to a distal end of the fourth portion opposite to the third portion, and a second gather elastic member attached along a boundary between the third portion and the fourth portion,
the standing gather being free of the gather elastic member in a region from the second gather elastic member toward the starting end,
the standing portion being provided with a gather waterproof sheet made of a waterproof resin film covering the third portion, fixedly attached to extend at least over an entire length in the front-back direction of the standing portion,
the gather waterproof sheet extending, from a position located within 5 mm from the second gather elastic member toward the starting end, to at least the starting end,
a fixed region between the gather waterproof sheet and the standing portion extending from a position located within 5 mm from an end of the gather waterproof sheet on a side of the second gather elastic member toward the starting end, to at least the starting end,
the standing gather further comprising a gather nonwoven fabric covering the third portion and the fourth portion, the fourth portion being free of the gather waterproof sheet and having fewer sheet layers than the third portion.

2. The disposable wearing article according to claim 1, further comprising an absorbent and
a liquid-permeable topsheet provided on a front side of the absorbent,
wherein the topsheet includes a topsheet extension extending out to either side in the width direction of the absorbent,
the topsheet extension including a topsheet fold-over portion folded back at the starting end onto the front side,
the topsheet fold-over portion extending from the starting end to a position located within ± 5 mm in the width direction relative to the end of the gather waterproof sheet on the side of the second gather elastic member, and being fixed to the gather waterproof sheet,
a fixed region between the topsheet fold-over portion and the gather waterproof sheet extending from a position located within 5 mm from an end of the topsheet fold-over portion on a side of the second gather elastic member toward the starting end, to at least the starting end.

3. The disposable wearing article according to claim 2, further comprising a back waterproof sheet made of a waterproof resin film provided on a back side of the absorbent,
wherein the back waterproof sheet includes a back waterproof sheet extension extending out to either side in the width direction of the absorbent,
the back waterproof sheet extension including a back waterproof sheet fold-over portion folded back at the starting end onto the front side,
the back waterproof sheet fold-over portion constituting the gather waterproof sheet.

4. The disposable wearing article according to claim 3, further comprising an outer nonwoven fabric provided on a back side of the absorbent,
wherein the outer nonwoven fabric includes an outer nonwoven extension extending out to either side in the width direction of the absorbent,
the outer nonwoven extension including an outer fold-over portion folded back at the starting end onto the front side,
the outer nonwoven fold-over portion extending from the starting end to a position located within ± 5 mm in the width direction relative to the end of the gather waterproof sheet on the side of the second gather elastic member, and being fixed to the gather waterproof sheet,
a fixed region between the outer nonwoven fold-over portion and the gather waterproof sheet extending from a position located within 5 mm from an end of the outer nonwoven fold-over portion on a side of the second gather elastic member toward the starting end, to at least the starting end.

5. The disposable wearing article according to any one of claims 1 to 4, wherein the third portion has a 80 to 150 mm bending resistance as determined by a method known as 45° cantilever method.

6. The disposable wearing article according to any one of claims 2 to 4, wherein the absorbent includes a narrower part with a reduced width in a middle part in the front-back direction,
the narrower part in a portion with a smallest width having a side edge distanced by 5 to 20 mm in the width direction from the starting end,
the starting end being distanced in the width direction from the second gather elastic member by 10 to 25 mm.
